(19)

**Europäisches Patentamt**

**European Patent Office**

**Office européen des brevets**

(11) **EP 1 961 378 A1**

(12) **EUROPEAN PATENT APPLICATION**

(43) Date of publication:
27.08.2008 Bulletin 2008/35

(51) Int Cl.:
*A61B 5/08* (2006.01)     *A61M 16/00* (2006.01)

(21) Application number: 08101753.5

(22) Date of filing: 19.02.2008

(84) Designated Contracting States:
AT BE BG CH CY CZ DE DK EE ES FI FR GB GR HR HU IE IS IT LI LT LU LV MC MT NL NO PL PT RO SE SI SK TR
Designated Extension States:
AL BA MK RS

(30) Priority: 23.02.2007 US 678454

(71) Applicant: GENERAL ELECTRIC COMPANY
Schenectady, NY 12345 (US)

(72) Inventors:
• Mitton, Michael Paul
Sun Prairie, WI 53590 (US)

• Tham, Robert Quinyew
Middleton, WI 53562 (US)
• Choncholas, Gary James
Madison, WI 53717 (US)
• Acker, Jaron Matthew
Madison, WI 53703 (US)
• Pinkert, John Raymond
Madison, WI 53711 (US)

(74) Representative: Illingworth-Law, William Illingworth
GE
London Patent Operation
15 John Adam Street
London
WC2N 6LU (GB)

(54) **Setting mandatory mechanical ventilation parameters based on patient physiology**

(57)     A method determines a subject's (12) natural exhalation time, when the subject's (12) natural exhalation flow ceases, and/or when the subject's (12) tidal volume is expired, and it sets the subject's (12) expiratory time based on the determination.

FIG. 2

**Description**

**FIELD OF INVENTION**

In general, the inventive arrangements relate to respiratory care, and more specifically, to improvements in controlling mandatory mechanical ventilation.

**BACKGROUND OF INVENTION**

Referring generally, when patients are medically unable to breathe on their own, mechanical, or forced, ventilators can sustain life by providing requisite pulmonary gas exchanges on behalf of the patients. Accordingly, modem ventilators usually include electronic and pneumatic control systems that control the pressure, flow rates, and/or volume of gases delivered to, and extracted from, patients needing medical respiratory assistance. Oftentimes, such control systems include a variety of knobs, dials, switches, and the like, for interfacing with treating clinicians, who support the patient's breathing by adjusting the afore-mentioned pressure, flow rates, and/or volume of the patient's pulmonary gas exchanges, particularly as the condition and/or status of the patient changes. Even today, however, such parameter adjustments, although highly desirable, remain challenging to control accurately, particularly using present-day arrangements and practices.

Referring now more specifically, ventilation is a complex process of delivering oxygen to, and removing carbon dioxide from, alveoli within patients' lungs. Thus, whenever a patient is ventilated, that patient becomes part of a complex, interactive system that is expected to promote adequate ventilation and gas exchange on behalf of the patient, eventually leading to the patient's stabilization, recovery, and ultimate ability to return to breathing normally and independently.

Not surprisingly, a wide variety of mechanical ventilators are available today. Most allow their operating clinicians to select and use several modes of ventilation, either individually and/or in various combinations, using various ventilator setting controls.

These mechanical ventilation modes are generally classified into one (1) of two (2) broad categories: a) patient-triggered ventilation, and b) machine-triggered ventilation, the latter of which is also commonly referred to as controlled mechanical ventilation (CMV). In patient-triggered ventilation, the patient determines some or all of the timing of the ventilation parameters, while in CMV, the operating clinician determines all of the timing of the ventilation parameters. Notably, the inventive arrangements described hereinout will be particularly relevant to CMV.

In recent years, mechanical ventilators have become increasingly sophisticated and complex, due, in large part, to recently-enhanced understandings of lung pathophysiology. Technology also continues to play a vital role. For example, many modem ventilators are now microprocessor-based and equipped with sensors that monitor patient pressure, flow rates, and/or volumes of gases, and then drive automated responses in response thereto. As a result, the ability to accurately sense and transduce, combined with computer technology, makes the interaction between clinicians, ventilators, and patients more effective than ever before.

Unfortunately, however, as ventilators become more complicated and offer more options, the number and risk of potentially dangerous clinical decisions increases as well. Thus, clinicians are often faced with expensive, sophisticated machines, yet few follow clear, concise, and/or consistent guidelines for maximal use thereof. As a result, setting, monitoring, and interpreting ventilator parameters can devolve into empirical judgment, leading to less than optimal treatment, even by well-intended practitioners.

Complicating matters ever further, ventilator support should be individually tailored for each patient's existing pathophysiology, rather than deploying a generalized approach for all patients with potentially disparate ventilation needs.

Pragmatically, the overall effectiveness of assisted ventilation will continue to ultimately depend on mechanical, technical, and physiological factors, with the clinician-ventilator-patient interface invariably continuing to play a key role. Accordingly, technology that demystifies these complex interactions and provides appropriate information to effectively ventilate patients is needed.

In accordance with the foregoing, it remains desirable to provide maximally effective mechanical ventilation parameters, particularly engaging clinicians to supply appropriate quantities and qualities of ventilator support to patients, customized for each individual patient's particular ventilated pathophysiology.

**SUMMARY OF INVENTION**

In one embodiment, a method of setting expiratory time in controlled mechanical ventilation comprises setting a subject's expiratory time based on the subject's natural exhalation time, when the subject's natural exhalation flow ceases, and/or when the subject's tidal volume is expired.

In another embodiment, a method of setting expiratory time in controlled mechanical ventilation comprises determining a subject's natural exhalation time, when the subject's natural exhalation flow ceases, and/or when said subject's tidal volume is expired, and setting the subject's expiratory time based on the determination.

In yet another embodiment, a device for use in controlled mechanical ventilation comprises a flow rate sensor configured to determine a subject's natural exhalation time, when the subject's natural exhalation flow ceases, and/or when the subject's tidal volume is expired, and base the subject's expiratory time on the determination.

## BRIEF DESCRIPTION OF SEVERAL VIEWS OF THE DRAWINGS

A clear conception of the advantages and features constituting inventive arrangements, and of various construction and operational aspects of typical mechanisms provided by such arrangements, are readily apparent by referring to the following illustrative, exemplary, representative, and/or non-limiting figures, which form an integral part of this specification, in which like numerals generally designate the same elements in the several views, and in which:

FIG. 1 depicts a front perspective view of a medical system comprising a ventilator;

FIG. 2 depicts a block diagram of a medical system providing ventilator support to a patient;

FIG. 3 depicts a block diagram of a ventilator providing ventilator support to the patient;

FIG. 4 depicts a flow diagram of the patient's inspiratory time ($T_I$), expiratory time ($T_E$), and natural exhalation time ($T_{EXH}$) for a single breath, particularly during controlled mechanical ventilation (CMV);

FIG. 5 depicts a flowchart of a simplified arrangement for setting the patient's expiratory time ($T_E$) based on the patient's natural exhalation time ($T_{EXH}$);

FIG. 6 depicts a flowchart of a simplified arrangement for setting the patient's expiratory time ($T_E$) based on when the patient's natural exhalation flow ceases;

FIG. 7 depicts a flowchart of a simplified arrangement for setting the patient's expiratory time ($T_E$) based on when the patient's tidal volume has expired;

FIG. 8 depicts a response curve of the patient's delivered inspiratory time ($dT_I$) and exhaled $CO_2$ levels ($F_{ET}CO_2$);

FIG. 9 depicts the delivered inspiratory time ($dT_I$) response curve of FIG. 8, graphically depicting an arrangement to identify the patient's optimal inspiratory time ($T_{I-OPTIMAL}$); and

FIG. 10 depicts a response curve of the patient's delivered inspiratory time ($dT_I$) and exhaled $VCO_2$ levels.

## DETAILED DESCRIPTION OF VARIOUS PREFERRED EMBODIMENTS

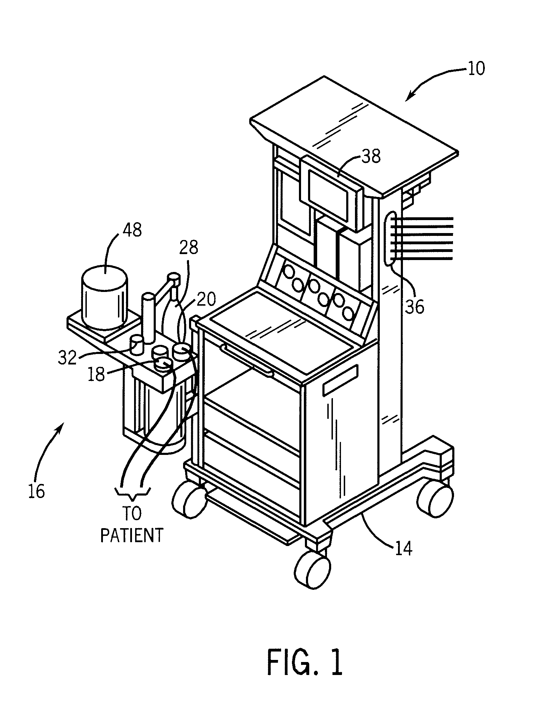

Referring now to the figures, and in particular to FIGS. 1-3, a medical system 10 is depicted for mechanically ventilating a patient 12. More specifically, an anesthesia machine 14 includes a ventilator 16, the latter having suitable connectors 18, 20 for connecting to an inspiratory branch 22 and expiratory branch 24 of a breathing circuit 26 leading to the patient 12. As will be subsequently elaborated upon, the ventilator 16 and breathing circuit 26 cooperate to provide breathing gases to the patient 12 via the inspiratory branch 22 and to receive gases expired by the patient 12 via the expiratory branch 24.

If desired, the ventilator 16 can also be provided with a bag 28 for manually bagging the patient 12. More specifically, the bag 28 can be filled with breathing gases and manually squeezed by a clinician (not shown) to provide appropriate breathing gases to the patient 12. Using this bag 28, or "bagging the patient," is often required and/or preferred by the clinicians, as it can enable them to manually and/or immediately control delivery of the breathing gases to the patient 12. Equally important, the clinician can sense conditions in the respiration and/or lungs 30 of the patient 12 according to the feel of the bag 28, and then accommodate for the same. While it can be difficult to accurately obtain this feedback while mechanically ventilating the patient 12 using the ventilator 16, it can also fatigue the clinician if the clinician is forced to bag the patient 12 for too long a period of time. Thus, the ventilator 16 can also provide a toggle 32 for switching and/or alternating between manual and automated ventilation.

In any event, the ventilator 16 can also receive inputs from sensors 34 associated with the patient 12 and/or ventilator 16 at a processing terminal 36 for subsequent processing thereof, and which can be displayed on a monitor 38, which can be provided by the medical system 10 and/or the like. Representative data received from the sensors 34 can include, for example, inspiratory time ($T_I$), expiratory time ($T_E$), natural exhalation time ($T_{EXH}$), respiratory rates ($f$), I:E ratios, positive end expiratory pressure (PEEP), fractional inspired oxygen ($F_IO_2$), fractional expired oxygen ($F_EO_2$), breathing gas flow (F), tidal volumes ($V_T$), temperatures (T), airway pressures ($P_{aw}$), arterial blood oxygen saturation levels ($S_aO_2$), blood pressure information (BP), pulse rates (PR), pulse oximetry levels ($S_pO_2$), exhaled $CO_2$ levels ($F_{ET}CO_2$), concentration of inspired inhalation anesthetic agent ($C_I$ agent), concentration of expired inhalation anesthetic agent ($C_E$ agent), arterial blood oxygen partial pressure ($P_aO_2$), arterial carbon dioxide partial pressure ($P_aCO_2$), and the like.

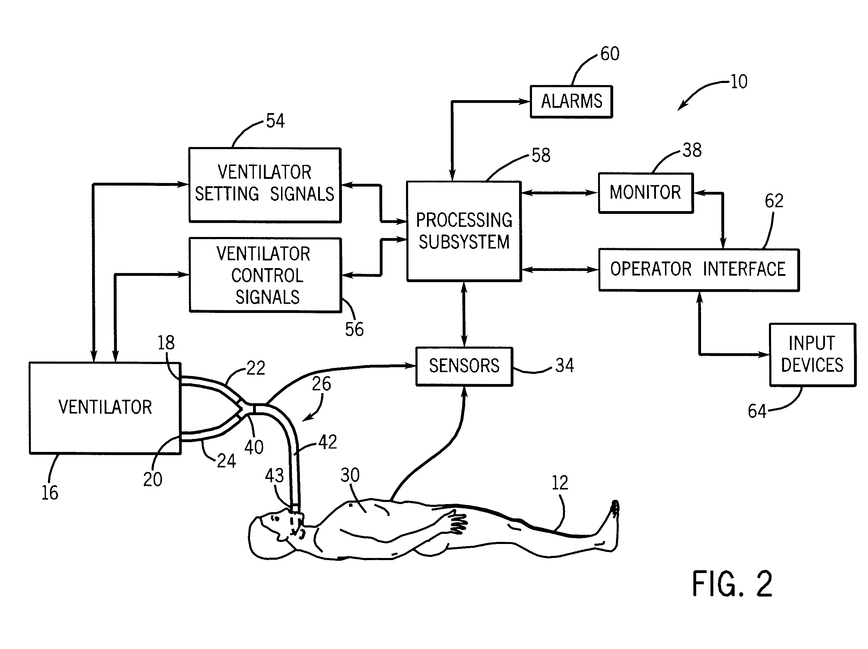

Referring now more specifically to FIG. 2, the ventilator 16 provides breathing gases to the patient 12 via the breathing circuit 26. Accordingly, the breathing circuit 26 typically includes the afore-mentioned inspiratory branch 22 and expiratory branch 24. Commonly, one end of each of the inspiratory branch 22 and expiratory branch 24 is connected to the ventilator 16, while the other ends thereof are usually connected to a Y-connector 40, which can then connect to the patient 12 through a patient branch 42, which can also include an interface 43 to secure the patient's 12 airways to the breathing circuit 26 and/or prevent gas leakage out thereof.

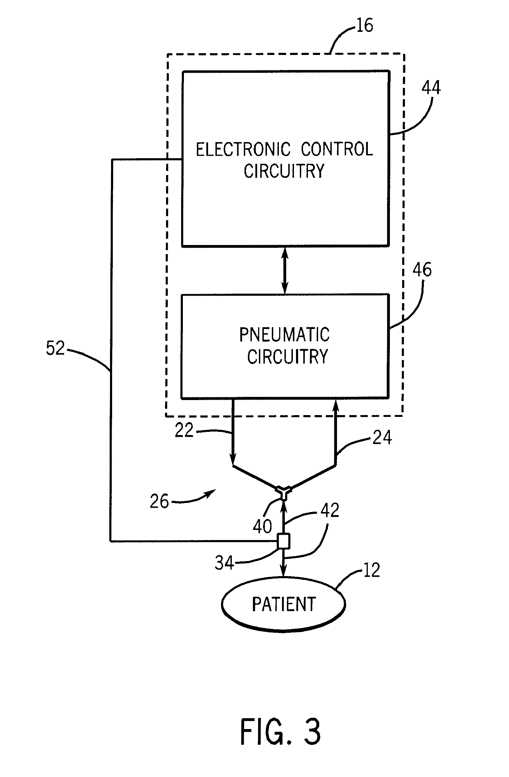

Referring now more specifically to FIG. 3, the ventilator 16 can also include electronic control circuitry 44 and/or pneumatic circuitry 46. More specifically, various pneumatic elements of the pneumatic circuitry 46 provide breathing gases to the lungs 30 of the patient 12 through the inspiratory branch 22 of the breathing circuit 26 during inhalation. Upon exhalation, the breathing gases are discharged from the lungs 30 of the patient 12 and into the expiratory branch 24 of the breathing circuit 26. This process can be iteratively enabled by the electronic control circuitry 44 and/or pneumatic circuitry 46 in the ventilator 16, which can establish various control parameters, such as the number of breaths per minute to administer to the patient 12, tidal volumes ($V_T$), maximum pressures, etc., that can characterize the mechanical ventilation that the ventilator 16 supplies to the patient 12. As such, the ventilator 16 may be microprocessor based and operable in conjunction with a suitable memory to control the pulmonary gas exchanges in the breathing circuit 26 connected to, and between, the patient 12 and ventilator 16.

Even more specifically, the various pneumatic elements of the pneumatic circuitry 46 usually comprise a source of pressurized gas (not shown), which can operate through a gas concentration subsystem (not shown) to provide the breathing gases to the lungs 30 of the patient 12. This pneumatic circuitry 46 may provide the breathing gases directly to the lungs 30 of the patient 12, as typical in a chronic and/or critical care application, or it may provide a driving gas to compress a bellows 48 (see FIG. 1) containing the breathing gases, which can, in turn, supply the breathing gases to the lungs 30 of the patient 12, as typical in an anesthesia application. In either event, the breathing gases iteratively pass from the inspiratory branch 22 to the Y-connector 40 and to the patient 12, and then back to the ventilator 16 via the Y-connector 40 and expiratory branch 24.

In the embodiment depicted in FIG. 3, one or more of the sensors 34, placed in the breathing circuit 26, can also provide feedback signals back to the electronic control circuitry 44 of the ventilator 16, particularly via a feedback loop 52. More specifically, a signal in the feedback loop 52 could be proportional, for example, to gas flows and/or airway pressures in the patient branch 42 leading to the lungs 30 of the patient 12. Inhaled and exhaled gas concentrations (such as, for example, oxygen $O_2$, carbon dioxide $CO_2$, nitrous oxide $N_2O$, and inhalation anesthetic agents), flow rates (including, for example, spirometry), and gas pressurization levels, etc., are also representative feedback signals that could be captured by the sensors 34, as can the time periods between when the ventilator 16 permits the patient 12 to inhale and exhale, as well as when the patient's 12 natural inspiratory and expiratory flows cease.

Accordingly, the electronic control circuitry 44 of the ventilator 16 can also control displaying numerical and/or graphical information from the breathing circuit 26 on the monitor 38 of the medical system 10 (see FIG. 1), as well as other patient 12 and/or system 10 parameters from other sensors 34 and/or the processing terminal 36 (see FIG. 1). In other embodiments, various components of which can also be integrated and/or separated, as needed and/or desired.

By techniques known in the art, the electronic control circuitry 44 can also coordinate and/or control, among other things, for example, other ventilator setting signals 54, ventilator control signals 56, and/or a processing subsystem 58, such as for receiving and processing signals, such as from the sensors 34, display signals for the monitor 38 and/or the like, alarms 60, and/or an operator interface 62, which can include one or more input devices 64, etc., all as needed and/or desired and interconnected appropriately (e.g., see FIG. 2). These components are functionally depicted for clarity, wherein various ones thereof can also be integrated and/or separated, as needed and/or desired. For further enhanced clarity, other functional components should also be well-understood but are not shown - e.g., one or more power supplies for the medical system 10 and/or anesthesia machine 14 and/or ventilator 16, etc. (not shown).

Now then, against this background, the inventive arrangements establish ventilation parameters according to patient physiology. These arrangements, to be now described, allow clinicians to control patient ventilation parameters throughout the patient's 12 respiratory cycle and enables ventilation treatments to be individually optimized for patients 12 subject to controlled mechanical ventilation (CMV).

To facilitate the following discussion, the following generalized and/or representative explanations and/or definitions may be referred to:

**1. $T_I$ is inspiratory time.**

More specifically, $T_I$ is the amount of time, measured in seconds, set on the ventilator 16 by the clinician, lasting from the beginning of the patient's 12 inspiration to the beginning of the patient's 12 expiration. Accordingly, $T_I$ is the patient's 12 inspiratory time.

Inspiratory times $T_I$ can be further broken down into a set inspiratory time $sT_I$, a delivered inspiratory time $dT_I$, and a measured inspiratory time $mT_I$. More specifically, the set inspiratory time $sT_I$ is the amount of time that the clinician sets on the ventilator 16 to deliver gases to the patient 12 during inspiration, while the delivered inspiratory time $dT_I$ is the amount of time that gases are actually allowed to be delivered to the patient 12 from the ventilator 16 during inspiration. Similarly, the measured inspiratory time $mT_I$ is the amount of time that the ventilator 16 measures for allowing gases to be delivered to the patient 12 during inspiration. Ideally, the set inspiratory time $sT_I$, delivered inspiratory time $dT_I$, and measured inspiratory time $mT_I$ are equal or substantially equal. However, if the clinician or ventilator 16 is searching for an optimal inspiratory time $T_{I-OPTIMAL}$, as elaborated upon below, then each of these inspiratory times $T_I$ may be different or slightly different. For example, the clinician and/or ventilator 16 may have established a set inspiratory time $sT_I$, yet the delivered inspiratory time $dT_I$ may deviate therefrom in the process of searching for, for example, the patient's 12 optimal inspiratory time $T_{I-OPTIMAL}$.

**2. $T_E$ is expiratory time.**

More specifically, $T_E$ is the amount of time, measured in seconds, set on the ventilator 16 by the clinician, lasting from the beginning of the patient's 12 expiration to the beginning of the patient's 12 inspiration. Accordingly, $T_E$ is the patient's 12 expiratory time.

Like inspiratory times $T_I$, expiratory times $T_E$ can also be further broken down into a set expiratory time $sT_E$, a delivered expiratory time $dT_E$, and a measured expiratory time $mT_E$. More specifically, the set expiratory time $sT_E$ is the amount of time that the clinician sets on the ventilator 16 to allow the patient 12 to exhale gases during expiration, while the delivered expiratory time $dT_E$ is the amount of time that gases are allowed to be exhaled by the patient 12 during expiration. Similarly, the measured expiratory time $mT_E$ is the amount of time that the ventilator 16 measures for having allowed the patient 12 to exhale gases during expiration. Ideally, the set expiratory time $sT_E$, delivered expiratory time $dT_E$, and measured expiratory time $mT_E$ are equal or substantially equal. However, if the clinician or ventilator 16 is searching for an optimal expiratory time $T_E$, as elaborated upon below, then each of these expiratory times $T_E$ may be different or slightly different. For example, the clinician and/or ventilator 16 may have established a set expiratory time $sT_E$, yet the delivered expiratory time $dT_E$ may deviate therefrom in the process of searching, for example, for the patient's 12 natural exhalation time $T_{EXH}$.

**3. I:E ratios are ratios between $T_I$ and $T_E$.**

More specifically, I:E ratios measure inspiratory times divided by expiratory times - i.e., $T_I / T_E$, which is commonly expressed as a ratio. Common I:E ratios are 1:2, meaning patients 12 may inhale for a certain period of time (x) and then exhale for twice as long (2x). However, since some patients 12 may have obstructed pathologies (e.g., chronic obstructive pulmonary disease (COPD)) and/or slower exhalation, requiring the clinician to set longer expiratory times $T_E$, I:E ratios can also be set at ratios closer to 1:3 and/or 1:4, particularly to provide the necessary expiratory time $T_E$ for a given patient 12 to fully exhale, although I:E ratios from 1:8 and 2:1 are also not uncommon, with common ventilators 16 providing 0.5 gradations therebetween.

**4. $T_{EXH}$ is natural exhalation time.**

More specifically, $T_{EXH}$ is the amount of time, measured in seconds, required for the patient's 12 natural exhalation flow to cease. Accordingly, $T_{EXH}$ is the patient's 12 natural exhalation time.

Oftentimes, the patient's 12 expiratory time $T_E$ does not equal the patient's 12 natural exhalation time $T_{EXH}$ - i.e., the patient's 12 expiratory time $T_E$, as set by the clinician on the ventilator 16, often does not coincide with the patient's 12 natural exhalation time $T_{EXH}$. Moreover, in accordance with many default settings on many ventilators 16, respiratory rates $f$ (see below) are commonly set between 6 - 10 breaths/minute and I:E ratios are commonly set at 1:2, resulting in many clinicians setting expiratory times $T_E$ between 4.0 - 6.6 seconds, as opposed to typical natural exhalation times $T_{EXH}$ being less than or equal to approximately 0.8 - 1.5 seconds. Several of the inventive arrangements, on the other hand, set the patient's 12 expiratory times $T_E$ approximately equal to the patient's 12 natural exhalation times $T_{EXH}$ (i.e., $2 * T_{EHX} \geq TE \geq T_{EXH}$).

If the clinician or ventilator 16 sets the patient's 12 expiratory time $T_E$ less than or equal to the patient's 12 natural exhalation time $T_{EXH}$, there can be inadequate time for the patient 12 to expel the gases in the patient's 12 lungs 30. This can result in stacking breaths in the patient's 12 lungs 30 (i.e., so-called "breath stacking"), thereby inadvertently and/or unknowingly elevating the patient's 12 lung pressure. Accordingly, several of the inventive arrangements set the patient's 12 expiratory time $T_E$ approximately equal to the patient's 12 natural exhalation time $T_{EXH}$, preferably with the patient's 12 expiratory time $T_E$ being set greater than or equal to the patient's 12 natural exhalation time $T_{EXH}$.

**5. PEEP is positive end expiratory pressure.**

More specifically, PEEP is the patient's 12 positive end expiratory pressure, often measured in $cmH_2O$. Accordingly, PEEP is the amount of pressure in the patient's 12 lungs 30 at the end of the patient's 12 expiratory time $T_E$, as controlled by the ventilator 16.

Like inspiratory times $T_I$ and expiratory times $T_E$, positive end expiratory pressure PEEP can also be further broken down into a set positive end expiratory pressure sPEEP, a measured positive end expiratory pressure mPEEP, and a delivered positive end expiratory pressure dPEEP. More specifically, the set positive end expiratory pressure sPEEP is the amount of pressure that the clinician sets on the ventilator 16 for the patient 12, while the measured positive end expiratory pressure mPEEP is the amount of pressure in the patient's 12 lungs 30 at the end of the patient's 12 expiratory time $T_E$. Similarly, the delivered positive end expiratory pressure dPEEP is the amount of pressure delivered by the ventilator to the patient 12. Usually, the set positive end expiratory pressure sPEEP, measured positive end expiratory pressure mPEEP, and delivered positive end expiratory pressure dPEEP are equal or substantially equal. However, the measured positive end expiratory pressure mPEEP can be greater than the set positive end expiratory pressure sPEEP when breath stacking, for example, occurs.

**6. $F_I0_2$ is fraction of inspired oxygen.**

More specifically, $F_IO_2$ is the concentration of oxygen in the patient's 12 inspiratory gas, often expressed as a fraction or percentage. Accordingly, $F_IO_2$ is the patient's 12 fraction of inspired oxygen.

**7. $F_EO_2$ is fraction of expired oxygen.**

More specifically, $F_EO_2$ is the concentration of oxygen in the patient's 12 expiratory gas, often expressed as a fraction or percentage. Accordingly, $F_EO_2$ is the patient's 12 fraction of expired oxygen.

**8. $f$ is respiratory rate.**

More specifically, $f$ is the patient's 12 respiratory rate, measured in breaths/minute, set on the ventilator 16 by the clinician.

**9. $V_T$ is tidal volume.**

More specifically, $V_T$ is the total volume of gases, measured in milliliters, delivered to the patient's 12 lungs 30 during inspiration. Accordingly, $V_T$ is the patient's 12 tidal volume.

Like inspiratory times $T_I$ and expiratory times $T_E$, tidal volumes $V_T$ can also be further broken down into a set tidal volume $sV_T$, a delivered tidal volume $dV_T$, and a measured tidal volume $mV_T$. More specifically, the set tidal volume $sV_T$ is the volume of gases that the clinician sets on the ventilator 16 to deliver gases to the patient 12 during inspiration, while the delivered tidal volume $dV_T$ is the volume of gases actually delivered to the patient 12 from the ventilator 16 during inspiration. Similarly, the measured tidal volume $mV_T$ is the volume of gases that the ventilator 16 measures for having delivered gases to the patient 12 during inspiration. Ideally, the set tidal volume $sV_T$, delivered tidal volume $dV_T$, and measured tidal volume $mV_T$ are equal or substantially equal. However, if the clinician or ventilator 16 is searching for a set optimal tidal volume $sV_T$, as elaborated upon below, then each of these set tidal volumes $sV_T$ may be different or slightly different.

**10. $F_{ET}CO_2$ is end tidal carbon dioxide $CO_2$.**

More specifically, $F_{ET}CO_2$ is the concentration of carbon dioxide $CO_2$ in the patient's 12 exhaled gas, often expressed as a fraction or percentage. Accordingly, $F_{ET}CO_2$ is the amount of carbon dioxide $CO_2$ exhaled by the patient 12 at the end of a given breath.

**11. $VCO_2$ is the volume of carbon dioxide $CO_2$ per breath.**

More specifically, $VCO_2$ is the volume of carbon dioxide $CO_2$ that the patient 12 exhales in a single breath. Accordingly, $VCO_2$ is the patient's 12 volume of $CO_2$ exhaled per breath.

Now then, clinicians usually begin ventilation by selecting an initial set tidal volume $sV_T$, respiratory rate $f$, and I:E ratio. The respiratory rate $f$ and I:E ratio usually determine the initial set inspiratory time $sT_I$ and initial set expiratory time $sT_E$ that the clinician sets on the ventilator 16. In other words, the actual set inspiratory time $sT_I$ and actual set expiratory time $sT_E$ that the clinician uses are usually determined in accordance with the following equations:

$$f = \frac{60}{sT_I + sT_E}$$

$$I:E = \frac{sT_I}{sT_E}$$

Moreover, the clinician usually makes these initial determinations based on generic rule-of-thumb settings, taking into account factors such as, for example, the patient's 12 age, weight, height, gender, geographical location, etc. Once the clinician makes these initial determinations, the inventive arrangements can now be appreciated.

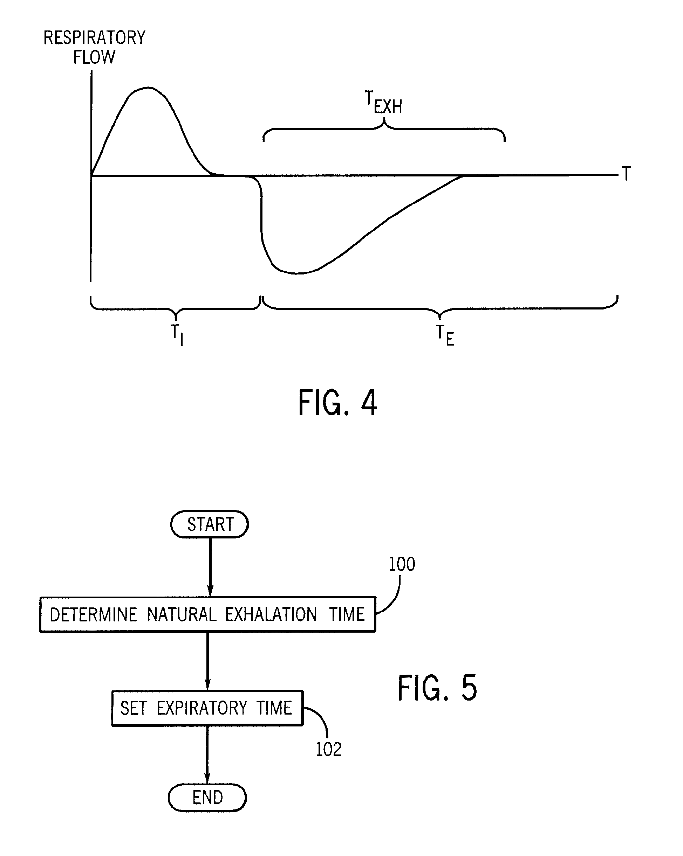

Referring now to FIG. 4, a graph of the relation between delivered inspiratory time $dT_I$, delivered expiratory time $dT_E$, and natural exhalation time $T_{EXH}$ is depicted for a single breathing cycle for a patient 12 undergoing controlled mechanical ventilation (CMV). As can be seen in the figure, the patient's 12 delivered expiratory time $dT_E$ is greater than the patient's 12 natural exhalation time $T_{EXH}$ as can be viewed by the measured expiratory time $mT_e$.

Referring now to FIG. 5, a flowchart depicts a simplied arrangement for setting the patient's 12 set expiratory time $sT_E$ based on the patient's 12 natural exhalation time $T_{EXH}$. More specifically, a method begins in a step 100, during which the patient's 12 natural exhalation time $T_{EXH}$ is determined. Preferably, the patient's 12 natural exhalation time $T_{EXH}$ is determined using the patient's 12 airway flow waveform, particularly when the first derivative thereof approaches zero, as is well-known in the art. Alternatively, other arrangements are also well-known in the art and can also be used to determine the patient's 12 natural exhalation time $T_{EXH}$ in step 100, such as, for example, airway flow analysis of the patient 12; tidal volume $V_T$ analysis of the patient 12; acoustic analysis of the patient 12; vibration analysis of the patient 12; airway pressure analysis $P_{aw}$ of the patient 12; capnographic morphology analysis of the patient 12; respiratory mechanics analysis of the patient 12; and/or thoracic excursion corresponding to gases exhaled from the lungs 30 of the patient 12 (e.g., imaging the patient 12, plethysmographic analysis of the patient 12, and/or electrical impedance tomography analysis of the patient, and/or the like), etc.

Thereafter, the patient's 12 natural exhalation time $T_{EXH}$ can be used to set the patient's 12 set expiratory time $sT_E$ on the ventilator 16. More specifically, the patient's 12 set expiratory time $sT_E$ can be set based on the patient's 12 natural exhalation time $T_{EXH}$, and, for example, set equal or substantively equal to the patient's 12 natural exhalation time $T_{EXH}$, as shown in a step 102 in FIG. 5, after which the method ends.

Now then, in accordance with the foregoing, the patient's 12 set expiratory time $sT_E$ is preferably set equal to, or slightly greater than, the patient's 12 natural exhalation time $T_{EXH}$.

If, however, the patient's 12 natural exhalation flow does not cease at the end of the patient's 12 ventilated set expiratory time $sT_E$, as set by the clinician and/or ventilator, then the clinician can increase the patient's 12 set expiratory time $sT_E$ until the patient's 12 natural exhalation flow ceases.

As previously noted, the patient's 12 spontaneous breathing is controlled by numerous reflexes that control the patient's 12 respiratory rates $f$ and tidal volumes $V_T$. Particularly during controlled mechanical ventilation (CMV), however, these reflexes are either obtunded and/or overwhelmed. In fact, one of the only aspects of ventilation that usually remains under the patient's 12 control is the patient's 12 natural exhalation time $T_{EXH}$, as required for a given volume, as previously elaborated upon. This is why it can be used to set the patient's 12 set expiratory time $sT_E$ on the ventilator 16 based thereon.

Now then, the inventive arrangements utilize the patient's 12 natural exhalation time $T_{EXH}$ and/or physiological parameters to determine and/or set the patient's 12 set expiratory time $sT_E$, set inspiratory time $sT_I$, and/or set tidal volume $sV_T$, either directly and/or indirectly. For example, the patient's 12 inspiratory time $T_I$ may be set directly, or may it be determined by the respiratory rate $f$ for a specific set expiratory time $sT_E$. Likewise, the patient's 12 set tidal volume $sV_T$ may also be set directly, or it may be determined by adjusting the patient's 12 inspiratory pressure ($P_{INSP}$) in, for example, pressure control ventilation (PCV). Adding the patient's 12 set inspiratory time $sT_I$ to the patient's 12 set expiratory time $sT_E$ results in a breath time that, when divided from 60 seconds, produces the patient's 12 respiratory rate $f$. Accordingly, the patient's 12 set inspiration time $sT_I$, set expiration time $sT_E$, and respiratory rate $f$ may not be whole numbers.

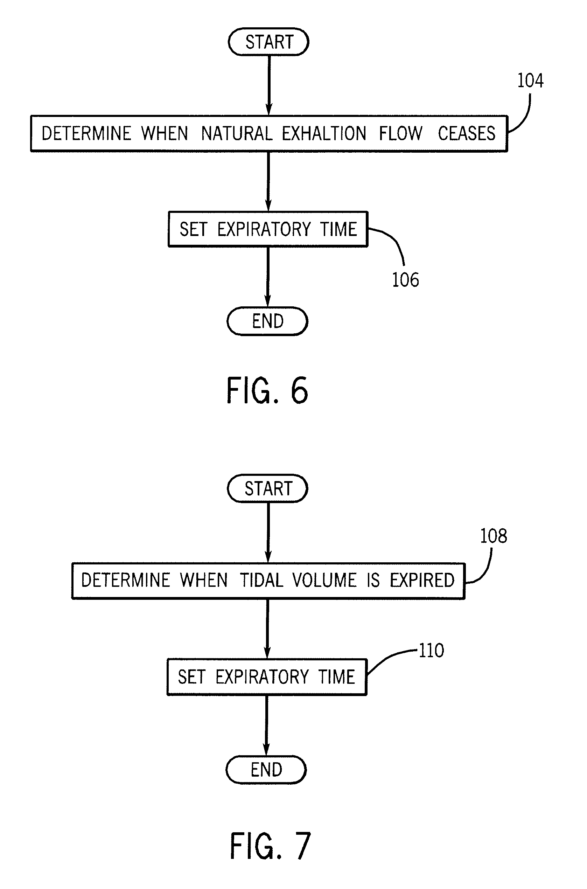

Referring now to FIG. 6, a flowchart depicts a simplied arrangement for setting the patient's 12 set expiratory time $sT_E$ based on when the patient's 12 natural exhalation flow ceases. More specifically, a method begins in a step 104, during which the patient's 12 natural exhalation flow cessation is determined. Preferably, the patient's 12 natural exhalation flow cessation is determined using the patient's 12 airway flow waveform, particularly when the first derivative thereof approaches zero, as is well-known in the art. Alternatively, other arrangements are also well-known in the art and can also be used to determine when the patient's 12 natural exhalation flow ceases.

Thereafter, the patient's 12 cessation of natural exhalation flow can be used to set the patient's 12 set expiratory time $sT_E$ on the ventilator 16. More specifically, the patient's 12 set expiratory time $sT_E$ can be set based on the patient's 12 cessation of natural exhalation flow, and, for example, set equal or substantively equal to when the patient's 12 natural exhalation flow ceases, as shown in a step 106 in FIG. 6, after which the method ends.

Referring now to FIG. 7, a flowchart depicts a simplied arrangement for setting the patient's 12 set expiratory time $sT_E$ based on when the patient's 12 tidal volume $V_T$ expires. More specifically, a method begins in a step 108, during which expiration of the patient's 12 tidal volume $V_T$ is determined. Preferably, the patient's 12 expiration of tidal volume $V_T$ is determined using a flow sensor. Alternatively, other arrangements are also well-known in the art and can also be used to determine when the patient's 12 tidal volume $V_T$ expires.

Thereafter, the patient's 12 expiration of tidal volume $V_T$ can be used to set the patient's 12 set expiratory time $sT_E$ on the ventilator 16. More specifically, the patient's 12 set expiratory time $sT_E$ can be set based on the patient's 12 expiration of tidal volume $V_T$, and, for example, set equal or substantively equal to when the patient's 12 tidal volume $V_T$ expires, as shown in a step 110 in FIG. 7, after which the method ends.

As previously indicated,

$$f = \frac{60}{sT_I + sT_E}$$

$$I : E = \frac{sT_I}{sT_E}$$

whereby knowing the patient's 12 respiratory rate $f$ and I:E ratio allows determining the patient's 12 set inspiratory time

$sT_I$ and set expiratory time $sT_E$, while knowing the patient's 12 set inspiratory time $sT_I$ and set expiratory time $sT_E$ conversely allows determining the patient's 12 respiratory rate $f$ and I:E ratio. Preferably, the clinician and/or the ventilator sets the patient's 12 respiratory rate $f$ and set expiratory time $sT_E$, for which the patient's 12 set inspiratory time $sT_I$ and I:E ratio can then be determined using the above equations.

While various mandatory mechanical ventilation modes can be used with the inventive techniques, volume guaranteed pressure control ventilation (i.e., PCV-VG), in particular, will be further described below as a representative example, as it has a decelerating flow profile based on the patient's natural exhalation in response to the ventilator delivered inspiratory pressure, and the set tidal volume $sV_T$ is guaranteed by the ventilator on a breath-to-breath basis. However, the inventive arrangements are also equally applicable to other pressure control ventilation (PCV) and/or other ventilator control ventilation (VCV) ventilator modes. In any event, several of the primary control settings on a typical ventilator 16 include controls for one or more of the following: set expiratory time $sT_E$, set inspiratory time $sT_I$, set tidal volumes $sV_T$, and/or fraction of inspired oxygen $F_IO_2$.

Now then, according to the patient's 12 physiological measurements in a steady state condition:

$$\dot{V}CO_2 = F_{ET}CO_2 * MV_A$$

wherein $\dot{V}CO_2$ is the volume of $CO_2$ per minute exhaled by the patient 12 and MV is the minute volume, which is a total volume exhaled per minute by the patient 12. As used in these expressions, a subscripted A indicates "alveolar," which is a part of the patient's 12 lungs 30 that participate in gas exchanges with the patient's 12 blood, in contrast to deadspace ($V_D$), such as the patient's 12 airway.

In this steady state condition and over a short duration, the patient's 12 blood reservoir is such that $\dot{V}CO_2$ is a constant (blood reservoir effects will be elaborated upon below), and, in accordance with this equation, as $MV_A$ increases, the patient's 12 end tidal carbon dioxide $F_{ET}CO_2$ decreases for a constant $\dot{V}CO_2$. Accordingly, substituting $MV_A = V_A * f$ yields the following:

$$\dot{V}CO_2 = F_{ET}CO_2 * V_A * f = F_{ET}CO_2 * V_A * \frac{60}{dT_I + dT_E}$$

$$V_T = V_A + V_D$$

Accordingly, the same $\dot{V}CO_2$ can be achieved by increasing the patient's 12 $V_A$ and/or decreasing the patient's 12 respiratory rate $f$. Decreasing the patient's 12 respiratory rate $f$ has the same effect as increasing the patient's 12 delivered inspiratory time $dT_I$ on the ventilator 16. In fact, numerous respiratory rate $f$ and delivered inspiratory time $dT_I$ combinations can result in equivalent or nearly equivalent $\dot{V}CO_2$ production. Accordingly, an optional combination is desired.

As previously described, the patient's 12 natural exhalation time $T_{EXH}$ measures the time period when the patient's 12 natural expiratory gas flow ceases during mechanical ventilation - i.e., the patient's 12 natural exhalation time $T_{EXH}$ comprises the duration of gas flow during the patient's 12 delivered expiratory time $dT_E$. A cessation of flow indicates that the patient's 12 lungs 30 are at their end expiratory lung volume (EELV). Continued gas exchange beyond EELV could become less efficient, largely as a result of the decreased volume of gases in the patient's 12 lungs 30 leading to reduced gas exchange gradient between the lung and the blood. As a result, initiating a new inspiration (i.e., the patient 12 starts a new breath) can be more efficient.

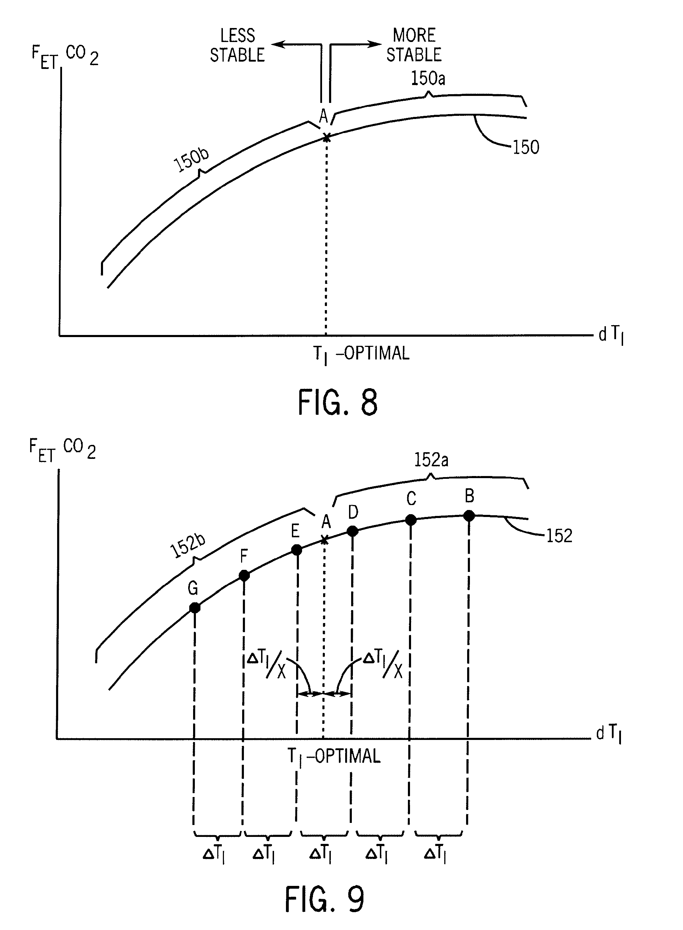

Referring now to FIG. 8, the clinician can also increase or decrease the patient's 12 set inspiratory time $sT_I$ on the ventilator 16 until the patient's 12 resulting end tidal carbon dioxide $F_{ET}CO_2$ is or becomes stable to changes in the patient's 12 delivered inspiratory time $dT_I$. More specifically, this will identify the patient's 12 optimal inspiratory time $T_{I-OPTIMAL}$. Preferably, the clinician and/or ventilator 16 will be able to determine this optimal inspiratory time $T_{I-OPTIMAL}$

within a few breaths of the patient 12 for any given inspiratory cycle. For example, when a stable end tidal carbon dioxide $F_{ET}CO_2$ is reached, then preferred equilibration of carbon dioxide $CO_2$ during a given delivered inspiratory time $dT_I$ can be achieved, as little or no more carbon dioxide $CO_2$ can be effectively extracted from the patient's 12 blood by further increasing the patient's 12 delivered inspiratory time $dT_I$. Accordingly, the patient's 12 optimal inspiratory time $T_{I-OPTIMAL}$ can then be ascertained and/or set.

More specifically, the patient's 12 end tidal carbon dioxide $F_{ET}CO_2$ can be considered stable or more stable at or after a point A on a $dT_I$ response curve 150 in the figure (e.g., see a first portion 150a of the $dT_I$ Response Curve 150) and non-stable or less stable or instable at or before that point A (e.g., see a second portion 150b of the $dT_I$ Response Curve 150). Accordingly, the point A on the $dT_I$ Response Curve 150 can be used to determine the patient's 12 optimal inspiratory time $T_{I-OPTIMAL}$, as indicated in the figure.

Physiologically, when the patient's 12 end tidal carbon dioxide $F_{ET}CO_2$ is equal to the patient's 12 capillary carbon dioxide $FcCO_2$, diffusion stops and carbon dioxide $CO_2$ extraction from the patient's 12 blood ceases. Ideally, the patient's 12 optimal inspiratory time $T_{I-OPTIMAL}$ is set where this diffusion becomes ineffective or stops. Otherwise, a smaller delivered inspiratory time $dT_I$ could suggest that additional carbon dioxide $CO_2$ could be effectively removed from the patient's 12 blood, while a larger delivered inspiratory time $dT_I$ could suggest that no additional carbon dioxide $CO_2$ could be effectively removed from the patient's 12 blood.

Preferably, finding the patient's 12 stable end tidal carbon dioxide $F_{ET}CO_2$ occurs without interference from the patient's 12 blood chemistry sequelae. A preferred technique for finding the patient's 12 stable end tidal carbon dioxide $F_{ET}CO_2$ can increase or decrease the patient's 12 inspiratory time $dT_I$, which may minimally disrupt the patient's 12 blood reservoir of carbon dioxide $CO_2$. Changes in the patient's 12 delivered inspiratory time $dT_I$ will affect how the patient's 12 blood buffers the patient's 12 carbon dioxide $CO_2$, and if that blood circulates back to the patient's 12 lungs 30 before the patient's 12 set inspiratory time $sT_I$ is optimized, then the patient's 12 end tidal carbon dioxide $F_{ET}CO_2$ will be different for a given inspiratory time $dT_I$. At that point, optimizing the patient's 12 set inspiratory time $sT_I$ may become a dynamic process. In any event, the time available to find the patient's 12 optimal inspiratory time $T_{I-OPTIMAL}$ may be approximately one (1) minute for an average adult patient 12.

One way to decrease the likelihood of interference from the patient's 12 blood chemistry sequelae is to change the patient's 12 delivered inspiratory time $dT_I$ for two (2) or more inspirations, and then use the patient's 12 resulting end tidal carbon dioxide $F_{ET}CO_2$ to extrapolate using an *apriori* function, such as an exponential function, by techniques known in the art.

For example, if the patient's 12 first end tidal carbon dioxide $F_{ET}CO_2$ was originally determined at a point B on a $dT_I$ Response Curve 152 in the figure, and then at a point C, and then at a point D, and then at a point E, and then at a point F, and then at a point G, and then so on, then the data points (e.g., points B - G) could be collected and a best fit $dT_I$ Response Curve 152 obtained; extrapolating as needed. Preferably, the $dT_I$ Response Curve 152 is piecewise continuous. For example, a first portion 152a of the $dT_I$ Response Curve 152 may comprise a stable horizontal or substantially horizontal portion (e.g., points B - D) while a second portion 152b thereof may comprise a polynomial portion (e.g., points E - G). Where this first portion 152a and second portion 152b of the $dT_I$ Response Curve 152 intersect (e.g., see point A on the $dT_I$ Response Curve 152) can be used to determine the patient's 12 optimal inspiratory time $T_{I-OPTIMAL}$, as indicated in the figure.

For example, referring now to FIG. 9, an arrangement to identify the patient's 12 optimal inspiratory time $T_{I-OPTIMAL}$ based on an iterative process will be described. More specifically, one preferred arrangement for determining an optimal inspiratory time $T_{I-OPTIMAL}$ collects $F_{ET}CO_2$ data in equal or substantially equal inspiratory time increments $\Delta T_I$. For example, if the patient's 12 first end tidal carbon dioxide $F_{ET}CO_2$ was originally determined to be within the first portion 152a of the $dT_I$ response curve 152 (e.g., see points B - D), then the clinician and/or ventilator 16 could decrease the patient's 12 delivered inspiratory times $dT_I$ until the patient's 12 end tidal carbon dioxide $F_{ET}CO_2$ readings were within the second portion 152b of the $dT_I$ response curve 152 (e.g., see points E - G).

For example, if the patient's 12 end tidal carbon dioxide $F_{ET}CO_2$ was originally determined to be at point C on the $dT_I$ response curve 152 (i.e., within the first portion 152a of the $dT_I$ response curve 152), then the patient's 12 delivered inspiratory time $dT_I$ could be decreased until the patient's 12 next end tidal carbon dioxide $F_{ET}CO_2$ was determined to be at point D on the $dT_I$ response curve 152, at which point the patient's 12 end tidal carbon dioxide $F_{ET}CO_2$ would still be determined to be within the first portion 152a of the $dT_I$ response curve 152. Accordingly, the patient's 12 delivered inspiratory time $dT_I$ could be decreased again until the patient's 12 next end tidal carbon dioxide $F_{ET}CO_2$ was determined to be at point E on the $dT_I$ response curve 152, at which point the patient's 12 end tidal carbon dioxide $F_{ET}CO_2$ would now be determined to be within the second portion 152b of the $dT_I$ response curve 152 (i.e., the patient's 12 end tidal carbon dioxide $F_{ET}CO_2$ would have dropped and thus not be at the patient's 12 optimal inspiratory time $T_{I-OPTIMAL}$). Accordingly, a smaller delivered inspiratory time increment $\Delta T_I / x$ could be made to determine when the patient's 12 end tidal carbon dioxide $F_{ET}CO_2$ was as at point A on the $dT_I$ response curve 152 - i.e., at the intersection of the first portion 152a of the $dT_I$ response curve 152 and the second portion 152b of the $dT_I$ response curve 152. In this iterative fashion, successively smaller delivered time increments and/or decrements $\Delta T_I$ are made to determine the patient's 12 optimal inspiratory time $T_{I\text{-OPTIMAL}}$, as indicated in the figure.

In like fashion, if the patient's 12 end tidal carbon dioxide $F_{ET}CO_2$ was originally determined to be at point F on the $dT_I$ response curve 152 (i.e., within the second portion 152b of the dTI response curve 152), then the patient's 12 delivered inspiratory time $dT_I$ could be increased until the patient's 12 next end tidal carbon dioxide $F_{ET}CO_2$ was determined to be at point E on the $dT_I$ response curve 152, at which point the patient's 12 end tidal carbon dioxide $F_{ET}CO_2$ would still be determined to be within the second portion 152b of the $dT_I$ response curve 152. Accordingly, the patient's 12 delivered inspiratory time $dT_I$ could be increased again until the patient's 12 next end tidal carbon dioxide $F_{ET}CO_2$ was determined to be at point D on the dTI response curve 152, at which point the patient's 12 end tidal carbon dioxide $F_{ET}CO_2$ would now be determined to be within the first portion 152a of the $dT_I$ response curve 152 (i.e., the patient's 12 end tidal carbon dioxide $F_{ET}CO_2$ would not have increased and thus not be at the patient's 12 optimal inspiratory time $T_{I\text{-OPTIMAL}}$). Accordingly, a smaller delivered inspiratory time decrement $\Delta T_I / x$ could be made to determine when the patient's 12 end tidal carbon dioxide $F_{ET}CO_2$ was as at point A on the $dT_I$ response curve 152 - i.e., at the intersection of the first portion 152a of the $dT_I$ response curve 152 and the second portion 152b of the $dT_I$ response curve 152. In this iterative fashion, successively smaller delivered time increments and/or decrements $\Delta T_I$ are again made to determine the patient's 12 optimal inspiratory time $T_{I\text{-OPTIMAL}}$, as indicated in the figure.

In addition, once the patient's 12 optimal inspiratory time $T_{I\text{-OPTIMAL}}$ is determined, it is realized this may be dynamic, by which the above arrangements can be repeated, as needed and/or desired.

Now then, a lower bound on the patient's 12 set inspiratory time $sT_I$ should be directly related to the minimal time required to deliver the patient's 12 minimal set tidal volume $sV_T$.

A lower bound for the patient's 12 set and delivered tidal volume $sV_T$, $dV_T$ should exceed $V_D$, preferably within a predetermined and/or clinician-selected safety margin. Preferably, a re-arrangement of the Enghoff-Bohr equation can be used to find $V_D$ or the following variation:

$$V_D = V_T - V_A = V_T - \frac{VCO_2}{F_{ET}CO_2}$$

After the patient's 12 end tidal carbon dioxide $F_{ET}CO_2$ is determined, then the patient's 12 set tidal volume $sV_T$ can be set accordingly, but it may not yet be set at an optimal value. Often, the clinician and/or ventilator 16 will attempt to determine this desired value. For example, the clinician may consider the desired value as the patient's 12 pre-induction end tidal carbon dioxide $F_{ET}CO_2$. The clinician can then adjust the patient's 12 set tidal volume $sV_T$ until the desired end tidal carbon dioxide $F_{ET}CO_2$ is achieved. Alternatively, or in conjunction therewith, a predetermined methodology can also be used to adjust the patient's 12 delivered tidal volume $dV_T$ until the desired end tidal carbon dioxide $F_{ET}CO_2$ is achieved. For example, such a methodology may use a linear method to achieve a desired end tidal carbon dioxide $F_{ET}CO_2$.

Preferably, the clinician can be presented with a dialog box on the monitor 38, for example (see FIG. 1), indicating the current and/or updated optimal ventilator 16 settings to be accepted or rejected. Preferably, the settings can be presented to the clinician in the dialog box for acceptance or rejection, who can then accept them, reject them, and/or alter them before accepting them. Alternatively, the settings can also be automatically accepted, without employing such a dialog box.

As previously indicated, different techniques can also be used to search for optimal settings for the ventilator 16. If desired, the delivered values can also be periodically altered to assess whether, for example, the settings are still optimal. Preferably, these alterations can follow one or more of the methodologies outlined above, and they can be determined based on a predetermined and/or clinician-selected time interval, on demand by the physiological, and/or determined by other control parameters, based, for example, on clinical events, such as changes in the patient's 12 end tidal carbon dioxide $F_{ET}CO_2$, or on clinical events such as changes in drug dosages, repositioning the patient, surgical events and the like. For example, the patient's 12 delivered inspiratory time $dT_I$ can vary about its current value set inspiratory time $sT_I$ and the resulting end tidal carbon dioxide $F_{ET}CO_2$ can be compared to the current end tidal carbon dioxide $F_{ET}CO_2$ to assess the optimality of the current settings. If, for example, a larger delivered inspiratory time $dT_I$ leads to a larger end tidal carbon dioxide $F_{ET}CO_2$, then the current set inspiratory time $sT_I$ could be too small.

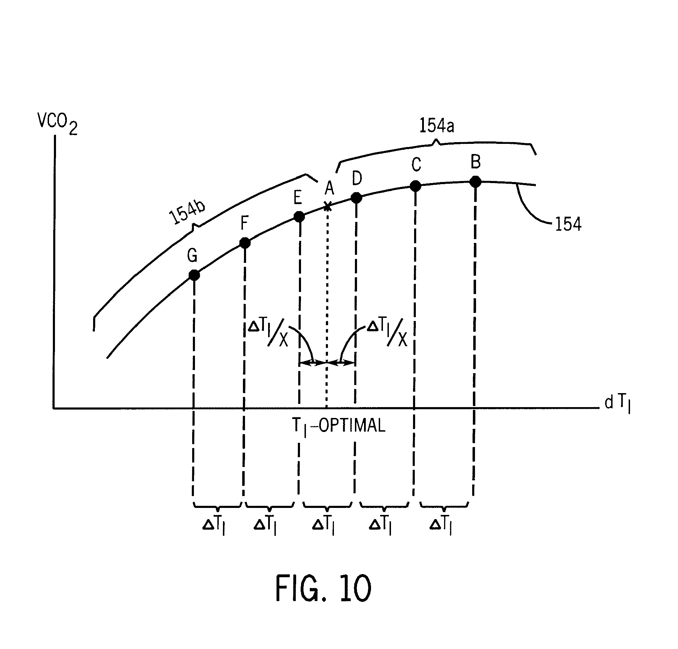

In an alternative embodiment, the $dT_I$ response curve 154 could be expressed in terms of $VCO_2$ instead of $F_{ET}CO_2$, as shown in FIG. 10. The morphology of the response curve 154 will be similar to that as shown in FIG. 9. Without loss of generality, the above techniques can be used to find $T_{I\text{-OPTIMAL}}$ utilizing $VCO_2$ as opposed to $F_{ET}CO_2$. The $VCO_2$ is equal to the inner product over one breath between a volume curve and a $CO_2$ curve. The flow and $CO_2$ curves should be synchronized in time.

One representative summary of potential inputs to, and outputs from, such a methodology is depicted below:

| Clinician Inputs | The patient's 12 age, weight, height, gender, location, and/or desired $F_{ET}CO_2$, etc. |
|---|---|
| Measured Inputs | End tidal carbon dioxide $F_{ET}CO_2$, flow wave data, etc. |
| Outputs | The patient's 12 set expiratory time $sT_E$, inspiratory time set, and/or set tidal volume $sV_T$ |

In addition, by more closely aligning the patient's 12 set expiratory time $sT_E$ and the patient's 12 natural exhalation time $T_{EXH}$ during mandatory mechanical ventilation, mean alveolar ventilation increases. In addition, there is additional optimal carbon dioxide $CO_2$ removal, improved oxygenation, and/or more anesthesia agent equilibration, whereby ventilated gas exchanges become more efficient with respect to use of lower set tidal volume $sV_T$ compared to conventional settings. Minute ventilations and respiratory resistance can be reduced, and reducing volumes can decrease the patient's 12 airway pressure $P_{aw}$ thereby reducing the risk of inadvertently over distending the lung.

In addition, the inventive arrangements facilitate ventilation for patients 12 with acute respiratory distress syndrome, and they can be used to improve usability during both single and double lung ventilations, as well transitions therebetween.

As a result of the foregoing, several of the inventive arrangements set the patient's 12 set expiratory time $sT_E$ equal to the time period between when the ventilator 16 permits the patient 12 to exhale and when the patient's 12 expiratory flow ceases - i.e., the patient's 12 natural exhalation time $T_{EXH}$. This facilitates the patient's 12 breathing by ensuring that ventilated airflows are appropriate for that patient 12 at that time in the treatment. In addition, methods of setting optimal patient inspired time $T_{I-OPTIMAL}$ and desired tidal volume are presented.

It should be readily apparent that this specification describes illustrative, exemplary, representative, and non-limiting embodiments of the inventive arrangements. Accordingly, the scope of the inventive arrangements are not limited to any of these embodiments. Rather, various details and features of the embodiments were disclosed as required. Thus, many changes and modifications-as readily apparent to those skilled in these arts-are within the scope of the inventive arrangements without departing from the spirit hereof, and the inventive arrangements are inclusive thereof. Accordingly, to apprise the public of the scope and spirit of the inventive arrangements, the following claims are made:

**Claims**

1. method of setting expiratory time in controlled mechanical ventilation, comprising:

   determining at least one or more of the following:

      a subject's (12) natural exhalation time;
      when said subject's (12) natural exhalation flow ceases; or
      when said subject's (12) tidal volume is expired; and

   setting said subject's (12) expiratory time based on said determination.

2. The method of Claim 1, wherein said determination is based, at least in part, on a flow rate sensor (34).

3. The method of Claim 2, wherein said flow rate sensor (34) is selected from a group consisting of a spirometer, anemometer, thermal anemometer, pneumotachometer, and ultrasound flow sensor.

4. The method of Claim 2, wherein said flow rate sensor (34) determines said subject's (12) breathing gas flow rate.

5. The method of any one of the preceding Claims, wherein said determination is based, at least in part, on effects of gas movement in said subject's (12) airways.

6. The method of Claim 5, wherein said effects are determined, at least in part, by one or more of the following:

   acoustic analysis of said subject (12);
   vibration analysis of said subject (12);
   airway pressure analysis of said subject (12);
   capnographic morphology analysis of said subject (12); and
   respiratory mechanics analysis of said subject (12).

7. The method of any one of the preceding Claims, wherein said determination is based, at least in part, on thoracic excursion of said subject (12) corresponding to said subject's (12) lung volume.

8. The method of Claim 7, wherein said thoracic excursion comprises one or more of the following:

   imaging said subject (12);
   plethysmographic analysis of said subject (12); and
   electrical impedance tomography analysis of said subject (12).

9. The method of any one of the preceding Claims, further comprising displaying at least one or more of the following on a monitor (38):

   said natural exhalation time;
   when said natural exhalation flow ceases;
   when said tidal volume is expired; or
   said expiratory time.

10. The method of Claim 9, wherein said monitor (38) is at least one of a handheld or portable device or both.

FIG. 1

FIG. 2

FIG. 3

RESPIRATORY
FLOW

$T_{EXH}$

T

$T_I$

$T_E$

## FIG. 4

START

DETERMINE NATURAL EXHALATION TIME

100

## FIG. 5

SET EXPIRATORY TIME

102

END

START

DETERMINE WHEN NATURAL EXHALTION FLOW CEASES — 104

SET EXPIRATORY TIME — 106

END

FIG. 6

START

DETERMINE WHEN TIDAL VOLUME IS EXPIRED — 108

SET EXPIRATORY TIME — 110

END

FIG. 7

FIG. 8

FIG. 9

FIG. 10

**European Patent Office**

**DECLARATION**

which under Rule 63 of the European Patent Convention shall be considered, for the purposes of subsequent proceedings, as the European search report

Application Number

EP 08 10 1753

| The Search Division considers that the present application, does not comply with the provisions of the EPC to such an extent that it is not possible to carry out a meaningful search into the state of the art on the basis of all claims | CLASSIFICATION OF THE APPLICATION (IPC) |
|---|---|

Reason:

CLASSIFICATION OF THE APPLICATION (IPC)

INV.
A61B5/08
A61M16/00

A meaningful search is not possible on the basis of all claims because all claims are directed to - Method for treatment of the human or animal body by therapy - Article 53 (c) EPC
The method claimed comprises the step of "determining a subject's natural exhalation time [..]", "setting said subject's expiratory time based on said determination. Consequently it is clear that the subject is connected to a mechanical ventilator, and that controlled mechanical ventilation is taking place on the subject's body. This is considered as a method of therapy excluded by Article 53(c) EPC.
Claims 2-10 are dependent on claim 1 and thus also do not fulfill the requirements of Article 53(c) EPC.

The applicant's attention is drawn to the fact that a search may be carried out during examination following a declaration of no search under Rule 63 EPC, should the problems which led to the declaration being issued be overcome (see EPC Guideline C-VI, 8.2).

-----

| Place of search | Date | Examiner |
|---|---|---|
| Munich | 30 May 2008 | Valfort, Cyril |

EPO FORM 1504 (P04F37)